Europäisches Patentamt

**European Patent Office**

Office européen des brevets

(11) Publication number: **0 119 633**
**A1**

(12) # EUROPEAN PATENT APPLICATION

(21) Application number: **84103059.6**

(22) Date of filing: **20.03.84**

(51) Int. Cl.³: **B 27 K 3/02,** B 27 K 3/10,
B 05 C 5/02

(30) Priority: **22.03.83 NZ 203654**

(71) Applicant: **DEVELOPMENT FINANCE CORPORATION
OF NEW ZEALAND, Development Finance Centre
Corner Grey and Featherston Streets, Wellington (NZ)**

(43) Date of publication of application: **26.09.84**
**Bulletin 84/39**

(72) Inventor: **Cross, David John, 13 Solly Place, Rotorua (NZ)**

(84) Designated Contracting States: **AT CH DE FR GB IT LI
SE**

(74) Representative: **Prüfer, Lutz H., Dipl.-Phys.,
Willroiderstrasse 8, D-8000 München 90 (DE)**

(54) **Method and apparatus for spreading liquids.**

(57) The invention relates to the spreading of a liquid onto
surfaces to be treated and to an apparatus used to spread
the liquid. The apparatus has a foaming element comprising
a porous body which is saturated with liquid and then sub-
jected to a gas under pressure which forces the liquid out of
the porous body in the form of a foam. The bubble sizes in
the foam are substantially uniform. In a preferred embodi-
ment the porous body is tubular with a hollow central core
and surrounding tubes injecting liquid into the body. The
gas, usually air, flows radially outwardly generating
bubbles. The method and device may be used to spread fun-
gicide to prevent sapstain on sawn timber.

0119633

METHOD AND APPARATUS FOR SPREADING LIQUIDS

This invention relates to a method and apparatus for evenly spreading fluids on surfaces. More particularly it relates to a method and apparatus for use in coating or impregnating flat surfaces but is not limited thereto.

In the sawmilling industry freshly milled timber can be subjected to unsightly staining owing to the presence of sapstain fungi. These fungi can be controlled by spreading fungicides on the wood surface immediately after sawing usually by dipping but sometimes by spraying. However, this adds to the costs of preparing sawn timber and it is desirable to be able to apply the minimum amount of fungicide to achieve cost effective treatment. There are also problems with adopting certain effective treating agents because of chemical corrosiveness or stability in the dip-spray systems.

It is known in the horticultural industry that insecticides and other protective sprays may be most effectively applied through the use of controlled droplet application. By this method droplets of substantially the same diameter are disbursed by spraying nozzles of a centrifugal design. This method improves targetting efficiency and avoids drift.

It has now unexpectedly been found that a variation of this principle can provide similar improvements in performance in the prophylactic treatment of sawn timber. By preparing a foam consisting of bubbles of substantially uniform size and spreading the foam evenly over a surface to be treated control of sapstain can be achieved using minimal quantities of chemical and with no wastage into the environment.

It is an object of this invention to go some way towards achieving these desiderata or at least.to offer the public a useful choice.

Accordingly the invention may be said broadly to consist in a method of applying a liquid to a surface which comprises preparing a foam of the said liquid having bubbles of a substantially uniform size and spreading said foam evenly over said surface.

Preferably said foam is created by injecting a liquid into a porous substrate held within a closed container, concurrently passing a gas under pressure through said substrate and releasing the foam thereby formed from said container.

Preferably said liquid contains a suspension, emulsion or a concentrated solution of a substance in water and said gas is air, carbon dioxide, nitrogen or other inert gaseous material.

referably said substance is a fungicide or fungicide and contact insecticide combination.

Preferably said porous substrate is a sintered glass substrate or other siliceous, ceramic/or synthetic plastic substrate.
metallic

Preferably said method includes the step of ejecting said liquid radially outwardly through a nozzle comprising a narrow slit or passageway running substantially parallel to the axis of a cylindrical container.

Alternatively said method includes the step of ejecting said liquid radially outwardly through a screen or mesh spaced from said porous substrate.

In another embodiment the invention may be said broadly to consist in an applicator for applying a liquid to a surface comprising:

a closed container,

a porous substrate within or associated with said closed container,

a nozzle out of said container,

means for injecting a liquid into said porous substrate, means for passing a gas through said substrate,

the arrangement being such that in use said liquid is injected into said porous substrate and said gas is passed thourgh said porous substrate to thereby create a foam, the gas pressure forcing said foam out of said substrate through said nozzle.

-4-

0119633

Preferably said substrate is a sintered glass or other similarly porous element having substantially uniform pore size.

Preferably said container, substrate and nozzle are all elongate integers so as to provide a relatively wide flow path of liquid out of said nozzle.

Preferably said means for injecting liquid into said porous substrate is a source of liquid communicating with the interior of the said substrate and extending substantially along the length thereof.

Preferably said source of liquid comprises a source of a suspension, emulsion or concentrated solution of a fungicide or contact insecticide.

Preferably said means for passing gas through said substrate comprises a source of gas under pressure arranged so that said substrate is between said source and said nozzle.

Preferably said source of gas is a source of air, carbon dioxide, nitrogen or other inert gaseous material.

In a second embodiment said source of gas comprises a central bore within said substrate and said liquid injecting means comprises one or more bores external to said central bore, all said bores being within said substrate.

0119633

Preferably said chamber is cylindrical.

Preferably said substrate has a regular geometric cross-section.

Preferably said cross-section is either square or circular.

In another embodiment the invention may be said broadly to consist in the combination of said applicator with an apparatus including means for conveying articles with surfaces to be coated in combination with said applicator.

Preferably said nozzle comprises a screen or mesh in the form of a roller surrounding said substrate.

Preferably said roller comprises a perforated cylindrical element rotating around a said substrate.

In another embodiment the invention may be said broadly to consist in an apparatus for generating a foam consisting of bubbles of a uniform size which apparatus comprises:

a porous substrate having substantially a uniform porosity at its periphery,

means for saturating said substrate with a liquid,

means for injecting a gas into said substrate,

the arrangement being such that in use gas injected into said substrate forces liquid saturating said substrate out of said substrate in a uniform bubble size.

0119633

The invention may be more fully understood by having reference to the accompanying drawings in which:

Figure 1 is an isometric projection (partly broken away) of a first embodiment of apparatus according to the invention.

Figure 2 is a schematic diagram illustrating an applicator according to the invention in combination with an apparatus for applying liquids to work pieces.

Figure 3 is an enlarged cross-sectional view of a foaming substrate.

Figure 4 is a cross-sectional view shown by the line IV - IV in Figure 1.

Figure 5 is an isometric view partly broken away of a second embodiment of the invention.

Figure 6 is an isometric view partly broken away of the other end of the embodiment illustrated in Figure 5.

Figure 7 is the view VII - VII shown in Figure 5.

0119633

Figure 8 is a schematic diagram illustrating an applicator in combination with a second embodiment of an apparatus for applying liquids to work pieces.

Figure 9 is a side elevational view (partially in section) of the applicator mounted in a conveying apparatus.

Figure 10 is an end elevational view of the applicator of Figure 9.

Figure 11 is the view XI – XI in Figure 9.

Figure 12 is a top plan view of the applicator shown in Figures 9 to 11 mounted in a preferred embodiment of a conveying apparatus.

Figure 13 is a side elevational view of the embodiment illustrated in Figure 12.

Figure 14 is an end elevational view of the embodiment illustrated in Figures 12 and 13.

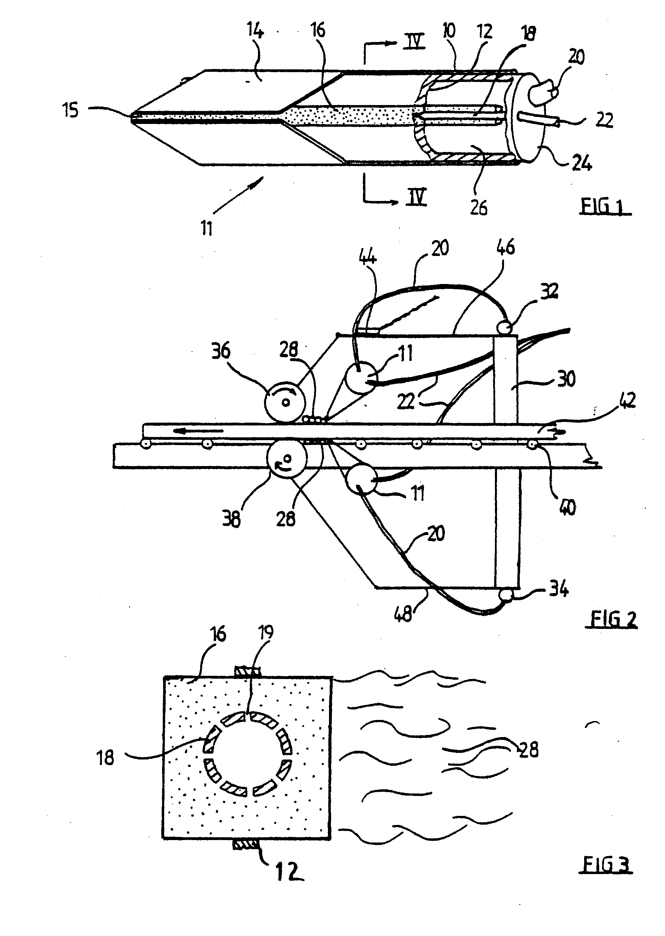

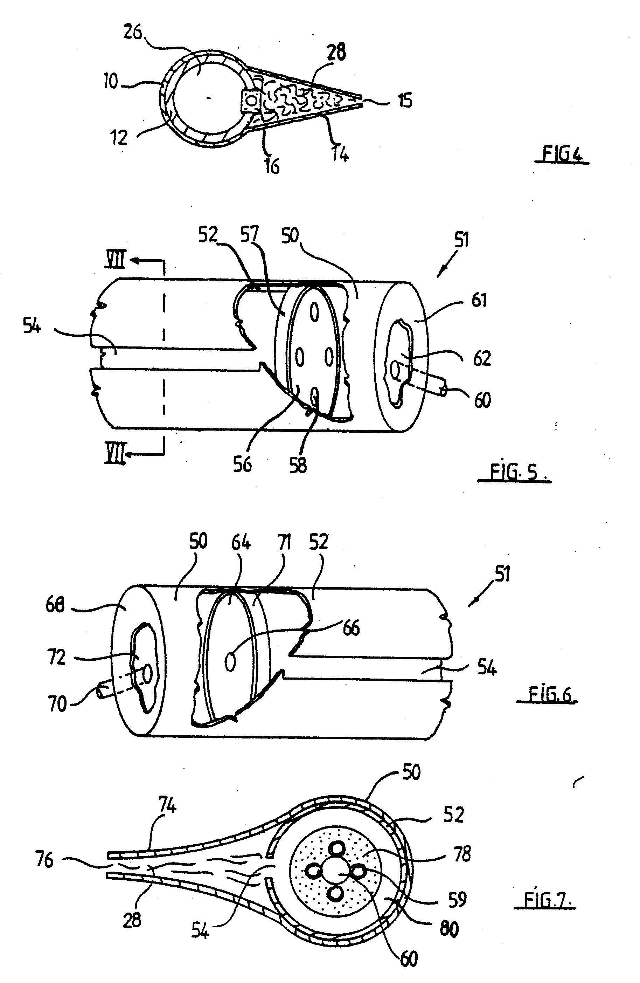

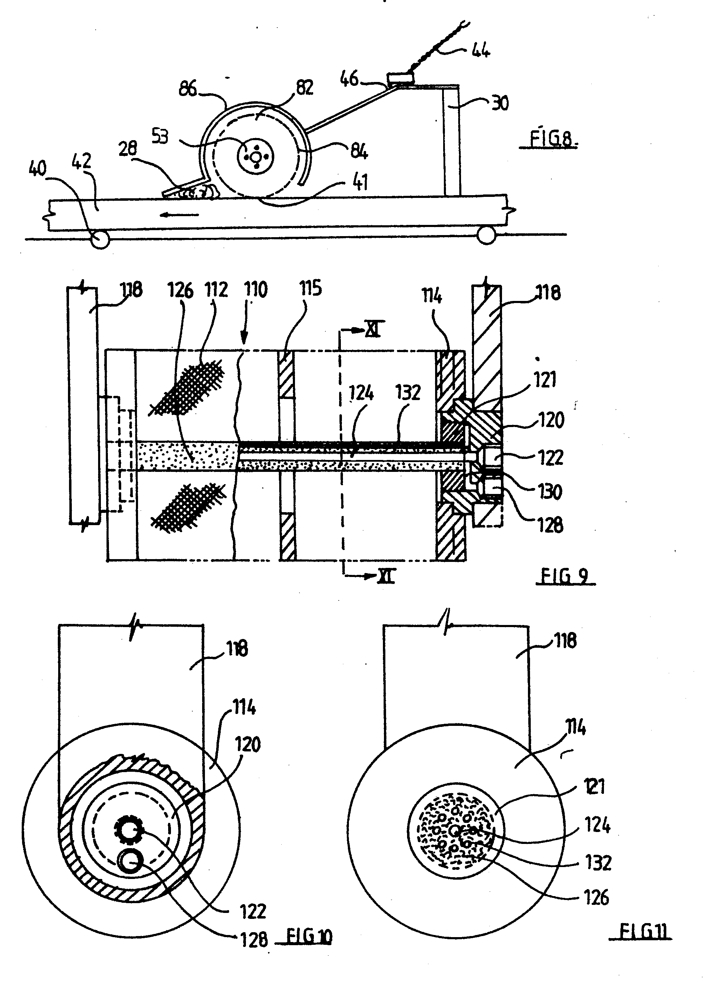

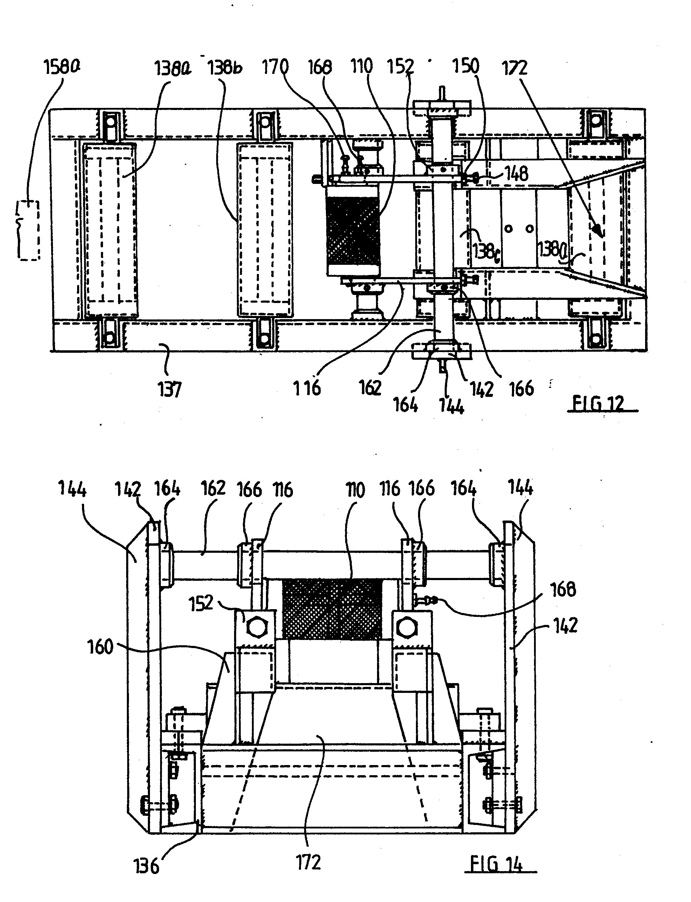

The liquid applicator 11 of the embodiment illustrated in Figures 1 to 4 comprises an outer casing 10, an inner casing 12 and nozzle plates 14 which come close to one another to form a nozzle outlet 15.  A foaming substrate 16 has a porous or perforated rod 18 at its centre.

An air hose 20 leads through end cap 24 to an interior chamber 26 within the applicator 11. Hose 22 is a source of liquid to rod 18. Although chamber 26 of the embodiment illustrated is circular in cross-section, it will be appreciated by those skilled in the art that other shapes can be employed. The applicator illustrated is intended to provide a wide swath of application and hence the nozzle opening 15 is an elongate opening.

Referring to Figure 3 the substrate 16 comprises in a preferred embodiment a sintered glass elongate element. Rod 18 can be either a rod made of some porous material or alternatively can be perforated with orifices 19.

Referring to Figure 2 the applicators 11 may be suspended within a frame work as illustrated to apply foam 28 to both the bottom and top of a work piece 42. The frame comprises a vertical frame member 30 and a roller track 40. Air hoses 20 supply compressed air from manifolds 32 and 34 to applicators 11.

A pair of rollers 36 and 38 are suspended from spring frame members 46 and 48 downstream from the nozzles of applicators 11. A sensor 44 capable of sensing whether arm 46 is urging roller 36 against a work piece 42 is also illustrated.

0119633

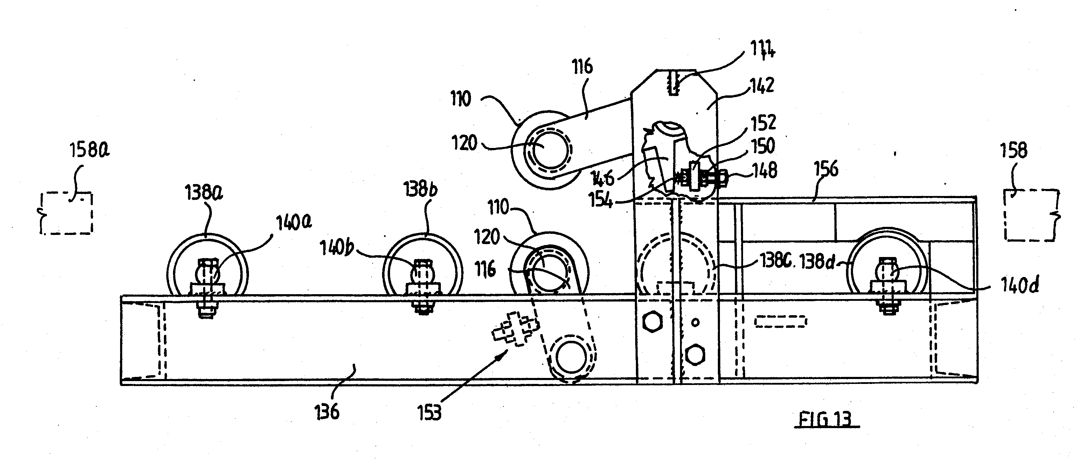

Turning to the second embodiment illustrated in Figures 5, 6 and 7 the applicator 51 comprises an outer casing 50 and an inner casing 52. Inner casing 52 has an outlet 54 along substantially its entire length. Through the end 56 illustrated in Figure 5 there are four openings 58 which are the end of liquid feeding tubes 59 within a sintered glass substrate 78. Inside end 61 there is formed a liquid chamber 62 which communicates via tube 60 with a source of liquid. A rim 57 surrounds the end 56 to form a seal with outer casing 50.

At the left hand end of applicator 51 as illustrated in Figure 6 through the end 64 there is provided an opening into the bore 66 of the sintered glass substrate 78. A chamber 72 is formed between the ends 64 and 68. An air tube 70 communicates with chamber 72. A rim 71 surrounds end 64 to form a seal with outer casing 50.

As seen from Figure 7 a pair of nozzle flanges 74 merge to a nozzle opening 76. Foam 28 is illustrated within the nozzle. A foam chamber 80 is created in the annular space between inner casing 52 and substrate 78. Chamber 80 is also formed between ends 56 and 64.

A still further embodiment is illustrated in Figure 8. A stationary applicator 53 is surrounded by rotatable drum 82 having a perforated or mesh surface 84. Applicator 53 is a porous tube whose construction is the same as that of porous tube 78 illustrated in Figure 7. It has a hollow central tube to

which air is fed and a series of surrounding tubes to which liquid is fed. Foam generated by air radiates outwardly through 360° from the outer surface of applicator 53 to fill the space inside mesh surface 84.

The foaming operation of the first embodiment will be described with reference to Figures 1, 3, and 4. Liquid under pressure fed through hose 22 enters rod 18 and the pressure forces the liquid either through the porous sides or the perforations 19 into the substrate 16 and substrate 16 becomes saturated.

At the same time air under pressure entering tube 20 passes through the substrate 16 causing the liquid which is saturated within the substrate 16 to foam. The bubbles of the foam are of a substantially uniform size and the foam 28 pours out nozzle opening 15.

In the embodiment illustrated in Figures 5 to 7 liquid is fed through tube 60 into chamber 62. Once chamber 62 is substantially full the pressure of the liquid causes it to flow along tubes 59 and to saturate the pores of substrate 78. From the other end of the applicator 51, air fed through hose 70 into chamber 72 passes through the central bore 66 and radially outward through the substrate 78 into the chamber 80 within inner casing 52. The foam 28 is then forced outwardly through opening 54 to the nozzle opening 76.

0119633

Turning to Figure 2 either the applicator 11 or the applicator 51 may be fitted into position to coat surfaces such as work pieces of wood on the apparatus. A work piece 42 is passed from right to left, as indicated by the direction of the arrow, on roller track 40. Rollers 36 and 38 are urged against the top and bottom surfaces respectively, of work piece 42 to evenly spread the foam 28 discharged from the nozzles 15 of the applicators 11. A sensor 44 senses when the two rollers come together after a work piece has passed the nip of the two rollers and automatically shuts off the air and liquid sources to applicator 11.

In another embodiment not illustrated there is an additional pair of applicators and rollers to treat the vertical sides of a work piece passing through a station.

In the embodiment illustrated in Figure 8 applicator 53 as illustrated is contained within a hollow perforated roller 82 in a configuration such that the roller 82 rotates concentrically around the applicator 53 while bearing on the surface of the work piece at contact point 41. Foam 28 from the applicator passes through the perforated surface 84 of the roller 82 and under the left hand lip of housing 86 onto the surface of work piece 42 being coated. The roller 82 in this embodiment is held within a housing 86 in such a way that the foam 28 ejected emerges only onto the surface being coated.

0119633

A still further embodiment of the apparatus will be described with reference to Figure 9 to 14.

The substrate for forming the foam is similar to that illustrated in Figures 5 to 7. However, the foam forming element is surrounded by a rotating mesh drum through which the bubbles pour as they are applied to the surface of the work piece as will be illustrated below.

Referring to Figure 9 the applicator comprises a drum 110. At either end of the drum is a rim 114 and in the centre is an annular reinforcing member 115 to which are fixed a stainless steel mesh screen 112. The rim portions 114 are rotatably fitted onto end body members 120. In the centre of drum 110 is a porous element 126 formed of a sintered glass of the like. Element 126 has a hollow center 124 and a series of hollow tubes 132 preferably symetrically disposed about central tube 124. Either end of element 126 is fitted into a collar 121 which itself is fitted into body member 120. Opening 122 communicates through body member 120 with central tube 124. Opening 128 communicates through annular opening 130 between body 120 and collar 121 into the open ends of tubes 132 inside porous element 126.

Referring to Figures 13 to 15 a pair of drums 110 are mounted one directly above another between pairs of brackets 116. Brackets 116 are fitted to bearing blocks 166 on fixed shaft 162. Shaft 162 is fixed at either end in blocks 164. Blocks 164 are each mounted on upright frame members 142. Frame members 142 are each reinforced by gusset members 144. At the bottom of upper bracket members 116 are a pair of abutment members 146. These abut against bolts 148 which are screwed through a pair of nuts 150 and 154 and a plate 152 which itself is fixed onto upper plate 156. It will be seen that by turning nut 148 brackets 116 are pivoted via abutment members 146 so that drums 110 are brought closer together or farther apart from one another.

Mounted along the top of side frame members 136 on top of surfaces 137 are a series of rollers 138 a, b, c and d in bearing blocks 140 a, b, c, and d. Lower roller 110 is adjusted by means of an adjustment bolt directly onto its mounting bracket as shown in ghost at 153 so that the upper surface of drum 110 is level with the tops of the drums 138 a, b, c, and d.

To the right of upright frame members 142 in Figures 12 and 13 there is formed a shute 172 for recieving work pieces 158 from other parts of the mill and feeding them between the pairs of rollers 110. Diagonal frame members 160 reinforce the framework.

In Figure 12 there is illustrated a pair of nipples 168 and 170 which respectively are connected to inlets 122 and 128 illustrated in Figure 10. Nipple 168 is connected to a source of air to feed tube 124 and nipple 170 is connected to a source of fluid such as an anti-sapstain suspension, to feed tubes 132 via annular space 130.

0119633

In operation adjustment bolt 153 is adjusted so that lower drum 110 is exactly level with the tops of adjacent drums 138 b and 138 c. A trial work piece 158 is placed between drums 110 and nuts 154 and 150 are loosened to allow bolt 148 to be adjusted so that both the upper and lower drum 110 just touch the work piece 158. A fine adjustment is then made to lower upper drum 110 so that the distance between the two drums 110 is slightly less than the anticipated minimum thickness of any work piece passing therebetween. It will be seen that in the event of a work piece 158 is thicker than this distance, brackets 116 of upper roller 110 can be pivoted in a clockwise direction when viewing Figure 13 to allow the work piece to pass through.

The air and liquid sources are then connected and turned onto the nipples 168 and 170 of the upper roller 110 and corresponding nipples on lower roller 110 and flow is commenced to create the foam which passes out through mesh 112 of each of the drums 110.

The foam generated will be adjusted to have a relatively high viscosity such as to minimise loss through dripping from the bottom of bottom roller 110. The rate of flow of gas and liquid into nipple 168 and 170 will be adjusted to ensure that most of the foam is wiped off the surfaces of rollers 110 contacting the workpiece 158 rather than being discharged at a rate too fast to be all taken up but the workpiece surface. If a lower viscosity fluid or higher pressures are used then a housing such as is illustrated in Figure 8 may be provided to cover rollers 110. A drip pan may be provided below lower roller 110.

0119633

In the embodiment illustrated all of the rollers 138 are idler rollers and the work piece is driven through the apparatus by means of its own momentum from a force applied earlier in the mill. A treated workpiece 158a is shown to the left in Figure 13. Alternatively, roller 138d may be a driven roller and would drive work piece 158 as it is passed over it. Both upper and lower surfaces of work piece 158 are coated by the foam passing through the screens of the two drums 110 and a fine smooth even coating is created on the work piece.

In the embodiments illustrated the work pieces are freshly sawn pieces of timber and the liquid being applied is a concentrated suspension of fungicide for the control of sapstain fungi. It will be appreciated by those skilled in the art that this is but a single application of the general principal of the invention. It may be used for the spreading of liquids for such diverse purposes as painting with primer or final coats or for a large range of other applications.

0119633

Claims

1. A method of applying a liquid to a surface which comprises preparing a foam of the said liquid having bubbles of a substantially uniform size and spreading said foam evenly over said surface.

2. A method according to claim 1 wherein said foam is created by injecting a liquid into a porous substrate held within a closed container, concurrently passing a gas under pressure through said substrate and releasing the foam thereby formed from said container.

3. A method according to claim 1 or 2 wherein said liquid contains a suspension, emulsion or a concentrated solution of a substance in water and said gas is air, carbon dioxide, nitrogen or other inert gaseous material.

4. A method according to any one of the preceding claims which includes the step of ejecting said liquid radially outward through a nozzle comprising a narrow slit or passageway running substantially parallel to the axis of a cylindrical container.

5. An applicator for applying a liquid to a surface comprising:
   a closed container,
   a porous substrate within or associated with said closed container,
   a nozzle out of said container,

0119633

means for injecting a liquid into said porous substrate, means for passing a gas through said substrate,

the arrangement being such that in use said liquid is injected into said porous substrate and said gas is passed through said porous substrate to thereby create a foam, the gas pressure forcing said foam out of said substrate through said nozzle.

6.   An applicator according to claim 5 wherein said substrate is a porous element having substantially uniform pore size.

7.   An applicator according to claim 6 wherein said porous element is sintered glass.

8.   An applicator according to any one of claims 5 to 7 wherein said container, substrate and nozzle are all elongate integers so as to provide a relatively wide flow path of liquid out of said nozzle.

9.   An applicator according to any one of claims 5 to 8 wherein said means for injecting liquid into said porous substrate is a source of liquid communicating with the interior of the said substrate and extending substantially along the length thereof.

10.   An applicator according to any one of claims 5 to 9 wherein said source of liquid comprises a source of a suspension, emulsion or concentrated solution of a fungicide or contact insecticide.

- 3 -

0119633

11. An applicator according to any one of claims 5 to 10 wherein said means for passing gas through said substrate comprises a source of gas under pressure arranged so that said substrate is between said source and said nozzle.

12. An applicator according to claim 11 wherein said source of gas comprising a central bore within said substrate and said liquid injecting means comprises one or more bores external to said central bore, all said bores being within said substrate.

13. An applicator according to any one of claims 5 to 12 in combination with conveying articles with surfaces to be coated thereby.

14. An applicator according to any one of claims 5 to 13 wherein said nozzle comprises a screen or mesh in the form of a roller surrounding said substrate.

15. An applicator according to claim 14 wherein said roller comprises a perforated cylindrical element rotating around a said substrate.

- 4 -

16. An apparatus for generating a foam consisting of bubbles of a uniform size which apparatus comprises:

a porous substrate having substantially a uniform porosity at its periphery,

means for saturating said substrate with a liquid,

means for injecting a gas into said substrate,

the arrangement being such that in use gas injected into said substrate forces liquid saturating said substrate out of said substrate in a uniform bubble size.

0119633

FIG 1

FIG 2

FIG 3

0119633

FIG.4

FIG.5.

FIG.6

FIG.7.

0119633

FIG 8.

FIG 9

FIG 10

FIG 11

FIG 12

FIG 14

FIG 13

0119633

**European Patent Office**

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US-A-4 158 076 (H.I. WALLSTEN)<br>* Column 8, lines 20-68; column 13, lines 42-46; claims * | 1,3,4 | B 27 K 3/02<br>B 27 K 3/10<br>B 05 C 5/02 |
| A | DE-A-2 049 005 (MASKINFABRIKKEN W. BRÜEL v/H. MICHAELSEN GASVAERKSBAKKEN)<br>* Claims * | 1,4 | |

-----

TECHNICAL FIELDS SEARCHED (Int. Cl. 3)

B 27 K
B 05 C
B 27 G

The present search report has been drawn up for all claims

| Place of search<br>THE HAGUE | Date of completion of the search<br>28-06-1984 | Examiner<br>FLETCHER A.S. |
|---|---|---|